# EUROPÄISCHE PATENTSCHRIFT

(11) **EP 4 108 333 B1**
(45) Veröffentlichungstag und Bekanntmachung des Hinweises auf die Patenterteilung: **07.02.2024**
(21) Anmeldenummer: 22179917.4
(22) Anmeldetag: 20.06.2022
(51) Int. Cl.: B01L 9/00, B01L 3/00, G01N 35/02, G01N 35/04, C12M 3/00, C12M 1/00, C12M 1/32

(54) **STAPEL-VORRICHTUNG FÜR MIKROTITERPLATTEN**
STACKING DEVICE FOR MICROTITER PLATES
DISPOSITIF D'EMPILAGE POUR MICROPLAQUES DE TITRAGE

(30) Priorität: 24.06.2021 CH 7372021
(43) Veröffentlichungstag der Anmeldung: 28.12.2022
(73) Patentinhaber: INTEGRA Biosciences AG, 7205 Zizers (CH)
(72) Erfinder: Städler, Andreas, 7012 Felsberg (CH); Tiedtke, Hans-Jürgen, 8634 Hombrechtikon (CH)
(74) Vertreter: Swisspat Riederer Hasler Patentanwälte AG

(56) Entgegenhaltungen:
- WO-A2-2006/017737
- US-A- 4 971 514
- US-A1- 2001 048 899
- US-B1- 6 193 102

## Beschreibung

### TECHNISCHES GEBIET DER ERFINDUNG

Die Erfindung betrifft eine Vorrichtung zum Aufnehmen, Stapeln und Entnehmen von Mikrotiterplatten gemäss dem Oberbegriff des Anspruchs 1, sowie ein System und ein Verfahren zum Hinzufügen oder Entnehmen von Mikrotiterplatten aus einem Stapel Mikrotiterplatten gemäss den Oberbegriffen der unabhängigen Ansprüche 11 und 13.

### HINTERGRUND DER ERFINDUNG

In der Laborarbeit werden eine grosse Anzahl von Mikrotiterplatten verwendet und für unterschiedliche Anwendung eingesetzt. Aus diesem Grund ist deren schnelle Verarbeitung von grosser Bedeutung was die Effizienz des gesamten Prozesses angeht. Die im Labor eingesetzten Mikrotiterplatten bestehen in der Regel aus einem Behälter und gegebenenfalls einem darüber angeordneten Deckel.

Eine schnelle Verarbeitung von Mikrotiterplatten wird unter anderem dadurch erzielt, dass die Mikrotiterplatten gestapelt werden. Die gestapelten Mikrotiterplatten werden mit Hilfe einer Vorrichtung einzeln weggeführt und ihrer vorgesehen Anwendung entsprechend verarbeitet. Dafür muss der Deckel der Mikrotiterplatte von dessen Behälter getrennt werden. Die Entfernung des Deckels einer Mikrotiterplatte, welche vom Stapel weggeführt wird, stellt für die Gestaltung eines effizienten Prozessablaufs unter Einhaltung der erforderlichen Arbeitsgenauigkeit eine technische Herausforderung dar. Dabei braucht die Trennung des Deckels der untersten Mikrotiterplatte vom Behälter der darüberliegenden Mikrotiterplatte eine zusätzliche Hilfsmassnahme.

Eine Vorrichtung zum Stapeln und einzeln Wegführen von Mikrotiterplatten ist in US 2015/0110690 A1 gezeigt. Die gezeigte Vorrichtung weist ein Gehäuse für die Aufnahme und das Stapeln von Mikrotiterplatten auf. Am unteren Ende des Gehäuses sind Halteelemente angebracht, welche jeweils einen Deckel einer Mikrotiterplatte halten können. In der Anfangsposition halten die Halteelemente den Deckel der untersten Mikrotiterplatte. Eine Transportvorrichtung hat einen direkten Zugriff auf die unterste Mikrotiterplatte im Stapel und kann durch die eigene Auf- und Abwärtsbewegung den Stapel heben oder senken, wobei die Abwärtsbewegung der Mikrotiterplatten durch die Halteelemente eingeschränkt sein kann. Die Halteelemente werden für den Wegtransport der untersten Mikrotiterplatte gelöst. Dadurch kommt die gesamte Gewichtskraft des Stapels auf der Hebevorrichtung zu liegen. Die Hebevorrichtung bewegt sich nun soweit nach unten bis die Haltelemente eine formschlüssige Verbindung mit dem Deckel der zweituntersten Mikrotiterplatte eingehen kann. Daraufhin liegt die ursprünglich unterste Mikrotiterplatte mit dem Behälter der ursprünglich zweituntersten Mikrotiterplatte auf der Hebevorrichtung. Der Behälter der ursprünglich zweituntersten Mikrotiterplatte muss von der Hebevorrichtung entfernt werden, nachdem die Hebevorrichtung mit der darauf platzierten Mikrotiterplatte die Vorrichtung zum Stapeln der Mikrotiterplatten verlassen hat. Dafür ist eine Greifvorrichtung vorgesehen, welche als ein separates Bauteil ausgeführt und seitlich an der Vorrichtung zum Stapeln der Mikrotiterplatten angeordnet ist. Damit muss eine separate Vorrichtung in Form einer Greifvorrichtung am Prozess beteiligt werden, wodurch die gesamte Vorrichtung grösser wird und die Mikrotiterplatten auf der Transportvorrichtung jeweils einen längeren Weg zurücklegen müssen. Zusätzlich zeigt sich in der Praxis, dass beim Erzeugen oder Lösen einer Formschlussverbindung zwischen einem Haltelement und einer Mikrotiterplatte die Mikrotiterplatte kleine ruckartige Bewegung durchführen kann. Diese sind bei einer gefüllten Mikrotiterplatte nicht gewünscht und idealerweise zu vermeiden.

In US 4 971 514 A ist eine Stapelvorrichtung zum Stapeln von Objekten, unter anderem Mikrotiterplatte, gezeigt. Die Stapelvorrichtung weist eine Klemmeinrichtung auf. Die Klemmeinrichtung weist zwei gegenüberliegend angeordnete Klemmbalken auf, welche durch die Bewegung in Richtung Mitte der Stapelvorrichtung eine Mikrotiterplatte fixieren. Die Bewegung der Klemmbalken wird durch einen Motor erzeugt, wobei die Klemmbalken an Druckfedern angebracht sind, die die erforderliche Klemmkraft erzeugen. Durch Verwendung von Druckfedern kann die Klemmkraft auf die Mikrotiterplatten unabhängig von deren Grösse angebracht werden. Die Klemmeinrichtung weist des Weiteren Bolzen auf, welche in Richtung Mitte der Stapelvorrichtung gerichtet und unterhalb eines durch die Klemmbalken fixierten Mikrotiterplatte angeordnet sind. Die Bolzen dienen zum Auffangen der Mikrotiterplatten, falls die Klemmbalken unerwartet versagen sollten. Die Bolzen sind unterhalb der Klemmbalken angeordnet.

### AUFGABE

Es ist deshalb eine Aufgabe der vorliegenden Erfindung, eine Vorrichtung zum Stapeln von Mikrotiterplatten vorzuschlagen, welche einen kompakten Aufbau aufweist und eine zuverlässige Funktionsweise ermöglicht. Die vorgeschlagene Vorrichtung soll eine hohe Zuverlässigkeit bei sich ändernden Rahmenbedingungen bieten.

### BESCHREIBUNG

Die obige Aufgabe wird gelöst mit einer Vorrichtung zum Aufnehmen, Stapeln und Entnehmen von Mikrotiterplatten mit den Merkmalen des Anspruchs 1.

Die Erfindung bezieht sich auf eine Vorrichtung zum Aufnehmen, Stapeln und Entnehmen von Mikrotiterplatten. Die Vorrichtung umfasst einen Turm für die Stapelung von Mikrotiterplatten und eine am unteren Ende des Turms angeordnete Haltevorrichtung, welche ein erstes und ein zweites Haltewerkzeug aufweist und vorzugsweise eine Mikrotiterplatte teilweise umschliesst. Eine Mikrotiterplatte umfasst einen Behälter und optional einen Deckel. Das erste Haltewerkzeug ist ausgebildet, eine Mikrotiterplatte formschlüssig zu halten. Das erste und zweite Haltewerkzeug sind zur Mitte des Turms hin bewegbar an der Haltevorrichtung angeordnet. Das zweite HaltewerkHaltewerkzeug ist ausgebildet, einen Behälter der Mikrotiterplatte kraftschlüssig zu fixieren. Das erste HaltewerkHaltewerkzeug ist in Stapelrichtung oberhalb des zweiten HaltewerkHaltewerkzeugs angeordnet.

Die erfindungsmässige Vorrichtung weist an ihrem unteren Ende eine Haltevorrichtung auf. Diese Haltevorrichtung wiederum umfasst zwei übereinander angeordnete HaltewerkHaltewerkzeuge, wobei das obere HaltewerkHaltewerkzeug einen Formschluss und das untere HaltewerkHaltewerkzeug einen Kraftschluss mit einer Mikrotiterplatte eingehen kann. Der Formschluss des ersten HaltewerkHaltewerkzeugs ist derart ausgelegt, dass er die Mikrotiterplatten an ihrer Bewegung nach unten hindert und als Ergebnis davon die Gewichtskraft aller in der Vorrichtung enthaltenen Mikrotiterplatten aufnehmen kann. Diese Gewichtskraft kann abhängig von der Anzahl an Mikrotiterplatten stark schwanken. Ein Vorteil der Formschluss-Verbindung ist, dass sie keine Anpassung bei sich ändernden Krafteinwirkungen braucht. Die Formschluss-Verbindung braucht lediglich derart stabil ausgelegt zu werden, dass sie die maximale Kraft aufnehmen kann. Falls die auf die Formschluss-Verbindung wirkende Kraft kleiner ist, wird die Funktionsweise der Formschluss-Verbindung nicht beeinträchtigt. Wenn das erste HaltewerkHaltewerkzeug eine Mikrotiterplatte fixiert, werden alle sich darüber befindenden Mikrotiterplatten auf jener Mikrotiterplatte aufliegen und entsprechend die auf das erste HaltewerkHaltewerkzeug wirkende Gewichtskraft erhöhen. Die Grösse dieser Gewichtskraft wird hauptsächlich von der Anzahl und dem Füllzustand der darüberliegenden Mikrotiterplatten bestimmt und ist wie bereits oben bemerkt im Betrieb grossen Schwankungen ausgesetzt.

Da das erste HaltewerkHaltewerkzeug die Gewichtskraft aller oberhalb von ihm liegenden Mikrotiterplatten aufnimmt, muss das zweite HaltewerkHaltewerkzeug lediglich eine Mikrotiterplatte fixieren und braucht nicht die Gewichtskraft weiterer Mikrotiterplatten zu tragen. Aus diesem Grund kann der Kraftschluss des zweiten HaltewerkHaltewerkzeugs gezielt auf diese Beanspruchung hin eingestellt werden, wodurch eine angepasste Dimensionierung des zweiten HaltewerkHaltewerkzeugs erzielt wird. Ein Vorteil der Kraftschluss-Verbindung, welche durch das zweite HaltewerkHaltewerkzeug mit der Mikrotiterplatte erzeugt wird, ergibt sich beim Abtransportieren oder Zuführen von Mikrotiterplatten. Die Transportiervorrichtung zum Abtransportieren oder Zuführen von Mikrotiterplatten kann die unterste Mikrotiterplatte aufnehmen oder abgeben, ohne dass die Mikrotiterplatte bei dieser Übergabe von der erfindungsgemässen Vorrichtung zur Transportiervorrichtung oder umgekehrt eine Bewegung durchführt.

Die Mikrotiterplatten werden in der Mitte des Turms gestapelt. Somit bildet die Bewegung der beiden Haltewerzeuge zur Mitte des Turms hin die direkteste und einfachste Möglichkeit zur Herstellung eines Kontakts zwischen den beiden Haltewerzeugen und einer Mikrotiterplatte. Da die beiden Haltewerzeuge lediglich eine translatorische Bewegung durchführen müssen, kann deren Aufbau konstruktiv einfach gestaltet sein. Zusätzlich erhöht die translatorische Bewegung nur in eine Richtung die Zuverlässigkeit der Bewegung der Haltewerzeuge.

Vorteilhafterweise ist am ersten HaltewerkHaltewerkzeug ein Greifer angebracht und der Greifer geeignet ist, die Mikrotiterplatte formschlüssig zu halten. Ein Greifer zeichnet sich durch eine längliche Form in Richtung seiner Auslenkung aus. Dabei ist der Greifer an einem seiner Enden angebracht, während sein anderes Ende zum Greifen oder Festhalten eines Gegenstandes dient. Der Greifer eignet sich für die Aufnahme von senkrecht in seiner Längsrichtung wirkenden Kräfte. In einer erfindungsgemässen Vorrichtung dient der Greifer am ersten HaltewerkHaltewerkzeug zur Aufnahme der Gewichtskraft der auf ihm liegenden Mikrotiterplatte. Aufgrund seiner länglichen Form erfüllt der Greifer seine Aufgabe mit einer minimalen Anforderung an Material und Platz. Vorzugsweise reicht der Greifer im ausgefahrenen Zustand etwa bis zur Seitenwand des Behälters einer im Turm aufgenommenen Mikrotiterplatte. Der Greifer muss der Seitenwand der Mikrotiterplatte derart nahe kommen, dass er unter die Deckelkante ragen und mit dem Deckel eine formschlüssige Verbindung eingehen kann. Dabei darf die Auslenkung des Greifers nicht derart gross sein, dass sich ein Kontakt zwischen dem Greifer und der Seitenwand des Behälters der Mikrotiterplatte ergibt.

Am ersten HaltewerkHaltewerkzeug sind vorzugsweise zwei Greifer angebracht. Zwei Greifer ermöglichen sowohl die von einem Greifer aufzunehmende Kraft zu halbieren und gleichzeitig eine grössere Stabilität beim Halten einer Mikrotiterplatte. Wenn der Schwerpunkt der Mikrotiterplatte etwa zwischen den beiden Greifern zu liegen kommt, kann die Mikrotiterplatte ohne Aufbringen einer grösseren Kraft nicht Kippen. Idealerweise umschliesst die Haltevorrichtung die unterste Mikrotiterplatte im Stapel derart, dass die auf den Greifern des ersten HaltewerkHaltewerkzeugs liegende Mikrotiterplatte keine Rotation durchführen kann und sich in einer stabilen Position befindet.

In einer bevorzugten Ausführungsform ist am zweiten HaltewerkHaltewerkzeug ein Bolzen angebracht und der Bolzen ist vorgesehen, den Behälter der Mikrotiterplatte zu fixieren. Die Mikrotiterplatte kann unter anderem einen Behälter und einen Deckel umfassen. Vorzugsweise ist das zweite HaltewerkHaltewerkzeug vorgesehen, den Behälter der Mikrotiterplatte zu fixieren. Der Bolzen ist ein zylinderförmiges Element, welches eine Höhe bzw. Länge aufweist, welche um Vielfaches grösser ist als sein Durchmesser. Der Bolzen ist vorgesehen derart angeordnet zu sein, dass er über eine seiner Kreisflächen mit der Mikrotiterplatte in Kontakt kommen kann. Dafür muss der Bolzen in seiner Längsrichtung bewegt werden.

Vorteilhafterweise sind am zweiten HaltewerkHaltewerkzeug zwei Bolzen angebracht. Bei Verwendung von zwei Bolzen am zweiten HaltewerkHaltewerkzeug wird die von einem Bolzen auf die Mikrotiterplatte auszuführende Kraft halbiert. Da die Kraft vom Bolzen auf die Mikrotiterplatte lediglich über die kreisrunde Fläche an einem Ende des Bolzens übertragen wird, ist der vom Bolzen auf die Mikrotiterplatte ausgeübte Druck proportional zu dieser Kraft und kann vergleichsweise auch hoch sein. Bei Aufteilung dieser Kraft auf zwei Bolzen wird der auf die Mikrotiterplatte ausgeübte Druck und damit die Beanspruchung der Mikrotiterplatte reduziert.

In einer weiteren bevorzugten Ausführungsform ist das erste HaltewerkHaltewerkzeug über ein erstes Federelement an der Haltevorrichtung angeordnet, wobei das erste Federelement vorzugsweise eine Druckfeder umfasst. Somit ist das erste HaltewerkHaltewerkzeug in seine Ausgangsposition gespannt und kann durch eine Betätigung eine Bewegung in Druckfeder-Richtung ausführen.

Vorteilhafterweise ist das zweite HaltewerkHaltewerkzeug über ein zweites Federelement an der Haltevorrichtung angeordnet, wobei das zweite Federelement vorzugsweise eine Druckfeder umfasst. Wie bereits oben erwähnt, ist das zweite HaltewerkHaltewerkzeug in diesem Fall in seine Ausgangsposition gespannt und nimmt infolge einer Betätigung eine Bewegung in Druckfeder-Richtung vor.

Vorzugsweise ist ein Schiebebalken an der Haltevorrichtung angebracht, welcher die Bewegungen der beiden Haltewerkzeuge auslöst. Somit kann durch die Bewegung des Schiebebalkens sowohl die Bewegung des ersten als auch die Bewegung des zweiten Haltewerkzeugs ausgelöst werden. Dies reduziert das zu bedienende Element zur Auslösung der Bewegung der Haltewerkzeuge auf nur einen Schiebebalken. Unter anderem kann dadurch ein kompakter Aufbau der HaltewerkHaltewerkzeuge realisiert werden, da diese keine Elemente für die Auslösung ihrer Bewegung aufweisen müssen.

Bevorzugt ist der Schiebebalken derart an der Haltevorrichtung angeordnet und steht mit den beiden HaltewerkHaltewerkzeugen in Verbindung, dass der Schiebebalken in einem ersten Schritt die Bewegung des ersten HaltewerkHaltewerkzeugs und in einem zweiten Schritt die Bewegung des zweiten HaltewerkHaltewerkzeugs auslöst. Aus dem Ruhezustand der HaltewerkHaltewerkzeuge wird mit einer ersten Bewegung des Schiebebalkens das erste HaltewerkHaltewerkzeug bewegt. Der Schiebebalken kann nach einem ersten Schritt wieder in die ursprüngliche Position geführt werden, wodurch das erste HaltewerkHaltewerkzeug ohne Bewegung des zweiten HaltewerkHaltewerkzeugs wieder in seine Ausgangsposition zurückkehrt. Die Bewegung des zweiten HaltewerkHaltewerkzeugs wird durch die Bewegung des Schiebebalkens in einem zweiten Schritt ausgelöst. Das heisst, dass bei Bewegung des zweiten HaltewerkHaltewerkzeugs das erste HaltewerkHaltewerkzeug sich in seiner ausgefahrenen Position befinden muss. Ausgehend von der Ruheposition beider HaltewerkHaltewerkzeuge wird zuerst eine Formschluss-Verbindung mittels des ersten HaltewerkHaltewerkzeugs erstellt. Anschliessend wird mit dem zweiten HaltewerkHaltewerkzeug eine Kraftschluss-Verbindung erzeugt, welche wiederum bei der Rückwärtsbewegung des Schiebebalkens als erste gelöst wird. Bei weiterer Bewegung des Schiebebalkens wird als letzter Schritt die Formschlussverbindung des ersten HaltewerkHaltewerkzeugs gelöst.

In einer weiteren bevorzugten Ausführungsform weist die Haltevorrichtung je zwei erste und zweite HaltewerkHaltewerkzeuge auf, welche jeweils gegenüberliegend zueinander angeordnet sind. Mit zwei gegenüberliegenden HaltewerkHaltewerkzeugen können die Mikrotiterplatten gleichzeitig von zwei Seiten fixiert werden. Dies führt zu einer zuverlässigeren Fixierung der Mikrotiterplatten. Idealerweise werden die gegenüberliegend angeordneten HaltewerkHaltewerkzeuge gleichzeitig bewegt. Zugleich erhöht sich auch die Anzahl an Angriffspunkte für die Kraft, welche vom HaltewerkHaltewerkzeug auf die Mikrotiterplatte ausgeübt wird, was wiederum zu einer kleineren Beanspruchung der Mikrotiterplatte führt.

Zwischen gestapelten Mikrotiterplatten kann je eine Zwischenplatte angeordnet sein. Die Zwischenplatte erhöht die zur Mikrotiterplatten-Ebene vertikale Distanz des Bereichs zum Einführen des Greifers der Haltevorrichtung. Damit werden die Erfordernisse an die Genauigkeit der Steuerung für die Bewegung der Greifer reduziert, wodurch die Steuerung der Greifer zuverlässig funktionieren und einfacher konstruiert werden kann. Nach Einführen der Greifer in eine Zwischenplatte nehmen die Greifer die Gewichtskraft der Zwischenplatte und aller der darüberliegenden Mikrotiterplatten auf.

Ein zweiter Aspekt der Erfindungsidee ist ein System mit einer erfindungsgemässen Vorrichtung zum Aufnehmen, Stapeln und Entnehmen von Mikrotiterplatten, einer Dispensier-Vorrichtung zum Befüllen von Mikrotiterplatten und einer Transportvorrichtung zur Entnahme und zum Zuführen von Mikrotiterplatten aus der oder zur Vorrichtung. Die Transportvorrichtung ist für den Transport der Mikrotiterplatte zwischen der Dispensiervorrichtung der Stapelvorrichtung zuständig. Die drei Vorrichtungen des Systems können ein einstückiges Bauteil bilden oder aber auch konstruktiv voneinander getrennt sein. Vorstellbar ist auch, dass nur zwei der drei Vorrichtungen miteinander konstruktiv verbunden sind. Idealerweise führt die Transportvorrichtung eine Bewegung in Richtung der Stapelrichtung der Mikrotiterplatten durch. Dies ermöglicht sowohl das Einführen der Mikrotiterplatten von unten in die erfindungsgemässe Vorrichtung als auch das Entnehmen dieser aus der erfindungsgemässen Vorrichtung.

In einer bevorzugten Ausführungsform umfasst das System eine erste und eine zweite Vorrichtung zum Aufnehmen, Stapeln und Entnehmen von Mikrotiterplatten, welche eine gemeinsame Transportvorrichtung aufweisen. Die zwei Vorrichtungen zum Aufnehmen, Stapeln und Entnehmen von Mikrotiterplatten teilen sich eine Transportvorrichtung. Idealerweise hat die erste Vorrichtung leere Mikrotiterplatte, welche nach deren Befüllung an die zweite Vorrichtung übergeben werden. Der Transport von der ersten Vorrichtung zu einer Dispensiervorrichtung und von dieser anschliessend zur zweiten Vorrichtung findet über die gemeinsame Transportvorrichtung statt.

Ein weiterer Aspekt der Erfindung betrifft ein Verfahren zum Hinzufügen von Mikrotiterplatten zu einem Stapel von Mikrotiterplatten oder Entnehmen von Mikrotiterplatten aus einem Stapel Mikrotiterplatten, wobei der Stapel sich in z-Richtung erstreckt. Bei welchem Verfahren wird eine Mikrotiterplatte jeweils von unten her zum Stapel hinzugefügt respektive die unterste Mikrotiterplatte des Stapels entnommen, wobei die unterste und zweitunterste Mikrotiterplatte jeweils durch in einer Ebene bewegliche und übereinander angeordnete Haltewerkzeuge gehalten werden, während eine in mindestens zwei Richtungen bewegliche Transportvorrichtung eine neue Mikrotiterplatte von unten dem Stapel hinzufügt oder die unterste Mikrotiterplatte dem Stapel entnimmt. Die Mikrotiterplatten werden in Z-Richtung gestapelt. Das obere Haltewerkzeug geht eine formschlüssige Verbindung und das untere Haltewerkzeug eine kraftschlüssige Verbindung mit der jeweiligen Mikrotiterplatte ein.

Mit dem vorgeschlagenen Verfahren kann aufgrund der Bewegung der Transportvorrichtung in eine Richtung eine Mikrotiterplatte aus einem Stapel von Mikrotiterplatten entnommen oder dieser hinzugefügt werden. Dies reduziert den Weg, den die Transportvorrichtung mit einer Mikrotiterplatte zurücklegen muss. Damit wird eine schnellere Verarbeitung der Mikrotiterplatte erreicht und die Gefahr einer Einwirkung von aussen auf die Mikrotiterplatte reduziert.

Vorzugsweise umfasst das Verfahren bei der Entnahme einer Mikrotiterplatte aus dem Stapel folgende Verfahrensschritte;
a) die Transportvorrichtung wird unter die unterste Mikrotiterplatte positioniert,
b) die kraftschlüssige Verbindung zwischen der untersten Mikrotiterplatte und des unteren Haltewerkzeugs wird gelöst, so dass die unterste Mikrotiterplatte auf der Transportvorrichtung aufliegt,
c) die Transportvorrichtung wird in Z-Richtung so weit nach oben bewegt, bis die auf der Transportvorrichtung aufliegende unterste Mikrotiterplatte mit der zweituntersten Mikrotiterplatte Kontakt hat,
d) die formschlüssige Verbindung zwischen der zweituntersten Mikrotiterplatte und des oberen Haltewerkzeugs wird gelöst, so dass der Stapel von Mikrotiterplatten auf der Transportvorrichtung aufliegt,
e) die Transportvorrichtung wird in Z-Richtung nach unten bewegt bis die drittunterste Mikrotiterplatte auf der Höhe des oberen Haltewerkzeugs zu liegen kommt,
f) die drittunterste Mikrotiterplatte wird durch das obere Haltewerkzeug formschlüssig gehalten wird,
g) die Transportvorrichtung wird in Z-Richtung weiter nach unten bewegt, bis die zweitunterste Mikrotiterplatte auf der Höhe des unteren Haltwerkzeugs zu liegen kommt,
h) sodass die zweitunterste Mikrotiterplatte durch das untere Haltewerkzeug kraftschlüssig in Eingriff genommen wird, und
i) die Transportvorrichtung mit der untersten Mikrotiterplatte weggeführt wird.

Das Verfahren beim Hinzufügen einer Mikrotiterplatte zum Stapel umfasst folgende Verfahrensschritte;
j) eine auf der Transportvorrichtung aufliegende Mikrotiterplatte wird unter die unterste Mikrotiterplatte im Stapel positioniert,
k) die kraftschlüssige Verbindung zwischen der untersten Mikrotiterplatte und dem unteren Haltewerkzeug wird gelöst, so dass die unterste Mikrotiterplatte auf der auf der Transportvorrichtung aufliegenden Mikrotiterplatte aufliegt,
1) die Transportvorrichtung wird in Z-Richtung so weit nach oben bewegt, bis die vormals unterste Mikrotiterplatte mit der darüberliegenden zweituntersten Mikrotiterplatte Kontakt hat,
m) die formschlüssige Verbindung zwischen der vormals zweituntersten Mikrotiterplatte und dem oberen Haltewerkzeug wird gelöst, so dass die vormals zweitunterste Mikrotiterplatte auf der untersten Mikrotiterplatte aufliegt,
n) die Transportvorrichtung wird in Z-Richtung nach oben bewegt, bis die vormals unterste Mikrotiterplatte des Stapels auf der Höhe des oberen Haltewerkzeugs zu liegen kommt,
o) die vormals unterste Mikrotiterplatte wird durch das obere Haltewerkzeug formschlüssig in Eingriff genommen,
p) die Transportvorrichtung wird in Z-Richtung nach unten bewegt, bis die auf der Transportvorrichtung liegende Mikrotiterplatte auf der Höhe des unteren Haltewerkzeugs zu liegen kommt und
q) die auf der Transportvorrichtung liegende Mikrotiterplatte durch das untere Haltewerkzeug kraftschlüssig in Eingriff genommen werden kann.

Die Verwendung einer kraftschlüssigen Verbindung ermöglicht einen direkten Zugang der Transportvorrichtung zur Mikrotiterplatte in der kraftschlüssigen Verbindung. Das heisst, dass die Transportvorrichtung bis zur Unterseite der aufzunehmenden Mikrotiterplatte bewegt werden und mit dieser in direkten Kontakt treten kann.

Im oben beschriebenen Verfahren hält das untere Haltewerkzeug, welches zur Erzeugung der kraftschlüssigen Verbindung mit einer Mikrotiterplatte vorgesehen ist, jeweils nur eine Mikrotiterplatte. Dadurch kann das untere Haltewerkzeug entsprechend dimensioniert werden.

Im Falle von Mikrotiterplatten, die je einen Behälter und einen Deckel umfassen, ist das Verfahren derart angepasst, dass bei der Entnahme einer Mikrotiterplatte aus dem Stapel
- in den Verfahrensschritten e) und f) der Deckel der zweituntersten Mikrotiterplatte durch das obere Haltewerkzeug gehalten wird, und
- in den Verfahrensschritten g) und h) der Behälter der zweituntersten Mikrotiterplatte durch das untere Haltewerkzeug gehalten wird, und
beim Hinzufügen einer Mikrotiterplatte zum Stapel
- die auf der Transportvorrichtung aufliegende Mikrotiterplatte einen Behälter und einen Deckel umfasst,
- in den Verfahrensschritten o) und p) der Deckel der auf der Transportvorrichtung liegende Mikrotiterplatte durch das obere Haltewerkzeug gehalten wird, und
- im Verfahrensschritt r) der Behälter der auf der Transportvorrichtung liegenden Mikrotiterplatte durch das untere Haltewerkzeug gehalten wird.

Bei einer Mikrotiterplatte mit einem Behälter und einem Deckel findet die Aufteilung der Mikrotiterplatte im Stapel zwischen dem Behälter und dem Deckel mit Hilfe der zwei Haltewerkzeuge statt. Obwohl das erste Haltewerkzeug mit einer formschlüssigen Verbindung sowohl den Behälter als auch den Deckel fixieren kann, ist es in der Regel vorgesehen, den Deckel der untersten Mikrotiterplatte im Stapel zu fixieren. Das zweite Haltewerkzeug ist dagegen vorgesehen, den Behälter der untersten Mikrotiterplatte zu halten. Somit ergibt sich eine spezifische Zuweisung der Haltewerkzeuge gegenüber den beiden Bestandteilen der Mikrotiterplatte.

Weitere Vorteile und Merkmale der Erfindung ergeben sich aus der nachfolgenden Beschreibung von Ausführungsbeispielen der Erfindung unter Bezugnahme auf schematische Darstellungen. Es zeigen in nicht massstabsgetreuer Darstellung:
- Figur 1:: eine dreidimensionale Ansicht eines Turms zum Stapeln von Mikrotiterplatten;
- Figur 2:: eine dreidimensionale Ansicht eines unteren Endes eines Turms zum Stapeln von Mikrotiterplatten mit einer daran angeordneten Haltevorrichtung;
- Figur 3:: einen Verfahrensablauf in Schritten I) - V) zum Entnehmen von Mikrotiterplatten aus einer erfindungsgemässen Vorrichtung;
- Figur 4:: einen Verfahrensablauf in Schritten I) - VI) zum Aufnehmen und Stapeln von Mikrotiterplatten in einer erfindungsgemässen Vorrichtung;
- Figur 5:: ein Querschnitt durch eine Haltevorrichtung mit übereinander angeordnetem Greifer und Bolzen;
- Figur 6:: eine dreidimensionale Ansicht einer Haltevorrichtung nach einer ersten Ausführungsform mit einem Schiebebalken;
- Figur 7:: ein Querschnitt durch die Haltevorrichtung aus Figur 6;
- Figur 8:: eine dreidimensionale Ansicht einer Haltevorrichtung nach einer zweiten Ausführungsform mit einer Kipphebel-Anordnung;
- Figur 9:: eine detaillierte Ansicht der Kipphebel-Anordnung der Haltevorrichtung aus Figur 8.

### DETAILIERTE BESCHREIBUNG DER FIGUREN

Im Folgenden stehen gleiche Bezugsziffern für gleiche oder funktionsgleiche Elemente (in unterschiedlichen Figuren). Ein zusätzlicher Apostroph weist auf ein mehrfaches Vorhandensein des selben Elements in einer Figur und dient gegebenenfalls zur Unterscheidung der identischen Elemente.

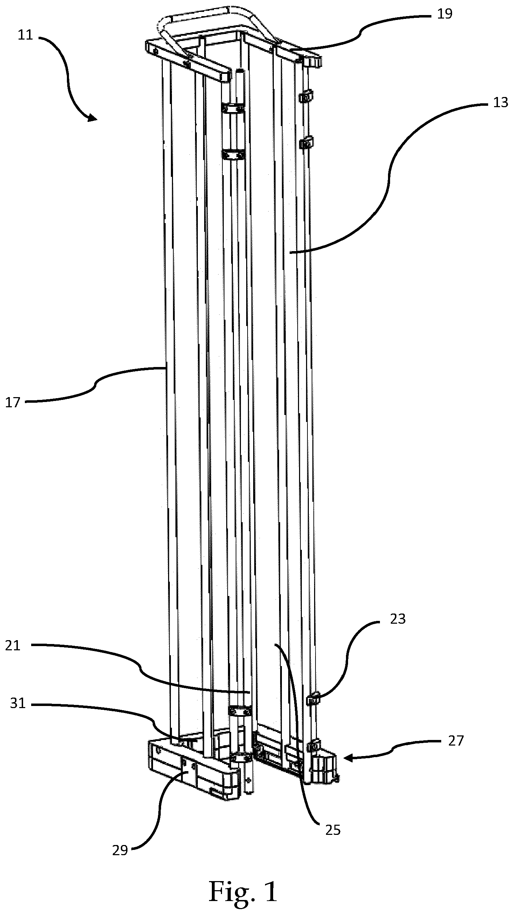

Figur 1 zeigt einen Turm 13 zum Stapeln von Mikrotiterplatten 15 (nicht in Figur gezeigt) im leeren Zustand. Der Turm 13 weist ein im Wesentlichen quaderförmiges Volumen auf, wobei die Längsseiten die Seitenwände bilden. Die Seitenwände wiederum sind durch eine Mehrzahl von in Längsrichtung des Turms gerichteten, parallel verlaufenden Stangen 17 gebildet. Der Querschnitt des Turms 13 entspricht in etwa der Fläche einer Mikrotiterplatte 15, so dass im Turm 13 Mikrotiterplatten 15 aufeinander gestapelt werden können und deren seitliche Bewegung durch die Stangen 17 eingeschränkt ist. An ihrem in Stapelrichtung oberen Ende sind alle Stangen 17 bis auf zwei durch ein U-förmiges Bauteil 19 zusammengehalten. Von den vier oberen Kanten des Turms sind drei Kanten durch das U-förmige Bauteil 19 abgedeckt. An der vierten und freien Kante sind die oberen Enden der zwei beweglichen Stangen 21, welche nicht am U-förmigen Bauteil 19 angebracht sind, angeordnet. Jene Seite des Turms 13, an welcher die zwei beweglichen Stangen 21 angeordnet sind, bildet die Vorderseite 25 des Turms. An den beweglichen Stangen sind Verbindungselemente 23 angebracht, welche die Stangen 21 mit ihren benachbarten Stangen verbinden. Die Verbindungselemente 23 ermöglichen es, dass die beweglichen Stangen 21 sich um ihre jeweils benachbarte Stange nach draussen drehen können. Durch die Drehbewegung der beiden beweglichen Stangen 21 ergibt sich eine offene Fläche auf der Vorderseite 25, durch welche das seitliche Einführen von Mikrotiterplatten ermöglicht ist.

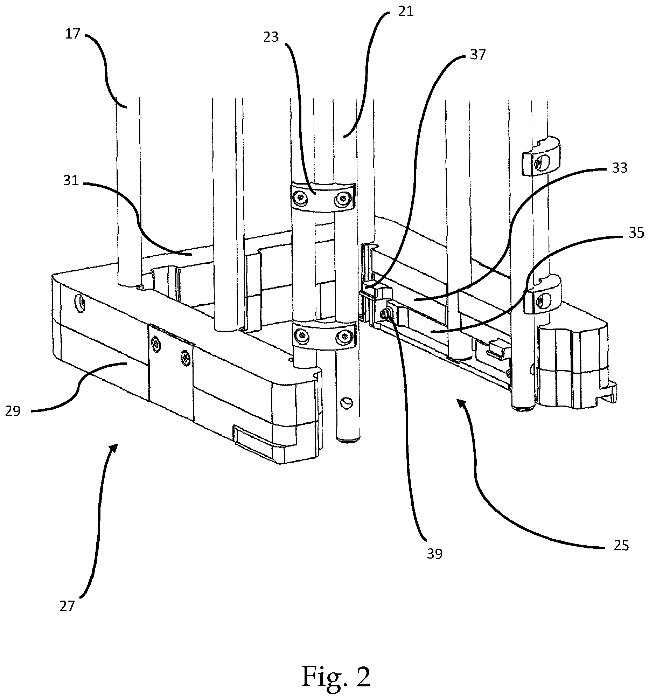

In Figur 2 ist das untere Ende des Turms 13 und die daran angebrachte Haltevorrichtung 27 gezeigt. Die Haltevorrichtung 27 deckt drei der vier unteren Kanten des Turms 13 ab. Die untere Kante der Vorderseite 25 ist wie die obere Kante frei und nicht abgedeckt. Die unteren Enden der beiden beweglichen Stangen 21 sind somit frei. Aufgrund der Verbindungselemente 23 zwischen einer beweglichen Stange 21 und ihr am nächsten liegende, fest angebrachte Stange 17 dreht sich die Stange 21 um die Achse jener Stange, mit welcher sie über das Verbindungselement 23 verbunden ist.

Die Haltevorrichtung 27 weist einen U-förmigen Aufbau auf, wobei zwei gegenüberliegend angeordnete Backen 29, 29' über ein Mittelstück 31 verbunden sind. Der Bereich zwischen den beiden Backen 29, 29' der Haltevorrichtung ist leer. Die leere Fläche zwischen den beiden Backen 29, 29' ist derart dimensioniert, dass eine für den Transport von Mikrotiterplatten vorgesehene Transportvorrichtung 30 (nicht gezeigt in Figur 1 und 2) durch diese Fläche in den Turm 13 geführt werden kann. Im Backen 29 der Haltevorrichtung 27 sind zwei HaltewerkHaltewerkzeuge 33,35 übereinander angeordnet. Das obere HaltewerkHaltewerkzeug 33 umfasst zwei Greifer 37. Die Greifer 37 sind nach innen des Turms 13 gerichtet. Die zwei Greifer 37 sind auf gleicher Höhe aber an unterschiedlichen Enden des oberen HaltewerkHaltewerkzeugs 33 angeordnet. Das obere HaltewerkHaltewerkzeug 33 ist vorgesehen, sich nach innen des Turms 13 und wieder nach aussen zu bewegen. In der Mitte der inneren Seite des oberen Haltewerkzeugs 33 ist eine Aussparung vorgesehen, durch welche eine Stange 17 geführt ist. Die Aussparung ist derart angeordnet, dass das Haltewerkzeug 33 sich nach innen und nach aussen des Turms 13 bewegen kann. Unterhalb des oberen Haltewerkzeugs 33 ist das untere Haltewerkzeug 35 angeordnet. Das untere Haltewerkzeug 35 weist zwei nach innen des Turms 13 gerichtete Bolzen 39 auf. In der Mitte der Innenseite des unteren Haltewerkzeugs 35 ist eine Aussparung vorgesehen, durch welche die gleiche Stange geführt ist wie diejenige durch die Aussparung im oberen Haltewerkzeug 33.

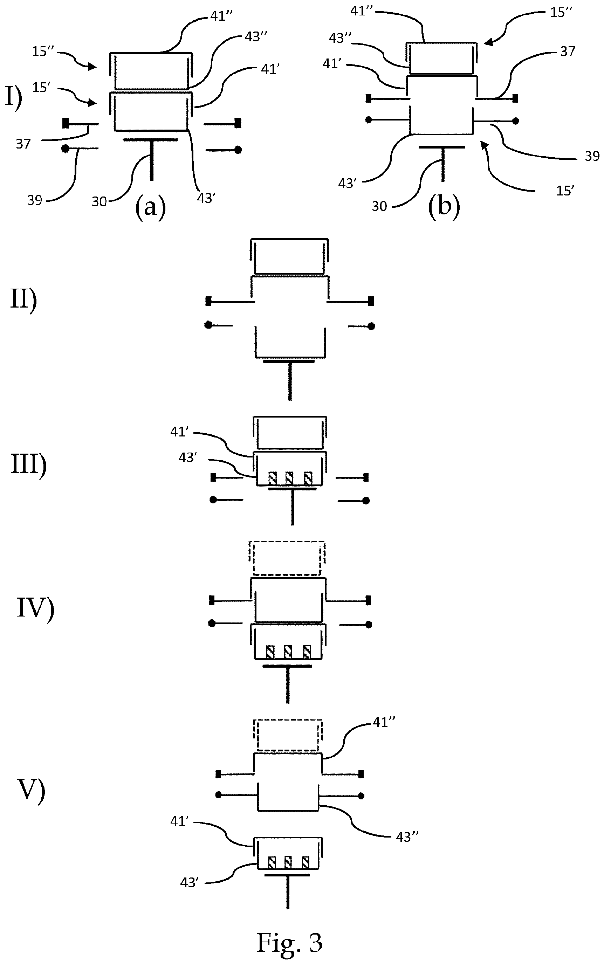

In Figur 3 ist die Arbeitsweise einer erfindungsgemässen Vorrichtungen in einzelnen Prozessschritten gezeigt. Im gezeigten Prozess wird die unterste Mikrotiterplatte 15' des im Turm 13 sich befindenden Stapels von Mikrotiterplatten 15 transportiert. Der Transport der Mikrotiterplatten 15 geschieht über eine Transportvorrichtung 30, welche einen Zugang zur jeweils untersten Mikrotiterplatte 15' im Stapel verfügt. Somit kann mit der hier gezeigten Vorrichtung stets die unterste Mikrotiterplatte 15' im Stapel transportiert werden. In der Ausgangslage ist die unterste Mikrotiterplatte 15' durch die Haltevorrichtung 27 fixiert. Dabei kann in einer ersten möglichen Ausgangslage (a) die unterste Mikrotiterplatte 15' mit ihrem Deckel 41' und Behälter 43' auf den Greifern 37 des oberen Haltewerkzeugs 33 liegen. In einer zweiten möglichen Ausgangslage (b) kommt der Deckel 41' der untersten Mikrotiterplatte 15' auf den Greifern 37 zu liegen und der Behälter 43' der untersten Mikrotiterplatte 15' wird durch die Bolzen 39 des unteren Haltewerkzeugs 35 fixiert. Während die Greifer 37 des oberen Haltewerkzeugs 33 eine Formschluss-Verbindung mit der Mikrotiterplatte 15 eingehen, kommt durch Fixieren der Mikrotiterplatte 15 mit den Bolzen 39 des unteren Haltewerkzeugs 35 eine Kraftschluss-Verbindung zustande. Die Ausgangslage ist im ersten Prozessschritt I) dargestellt.

Ausgehend von der ersten Ausgangslage (a) wird die Transportvorrichtung 30 derart unter der untersten Mikrotiterplatte 15' platziert, dass die Transportvorrichtung 30 die gesamte Gewichtskraft des Mikrotiterplatten-Stapels aufnehmen kann. Die Formschluss-Verbindung durch die Greifer 37 wird daraufhin gelöst, so dass die unterste Mikrotiterplatte 15' mit der Transportvorrichtung 30 nach unten bewegt werden kann. Die Greifer 37 werden nach innen bewegt, sobald die untere Kante des Behälters 43' der untersten Mikrotiterplatte 15' unterhalb der Greifer 37 liegt. Dadurch kommt der Deckel 41' der untersten Mikrotiterplatte 15' auf den Greifern 37 zu liegen, während der Behälter 43' der untersten Mikrotiterplatte 15' auf der Transportvorrichtung 30 liegt und für die Weiterbehandlung weggeführt werden kann, siehe Prozessschritt II).

In der zweiten Ausgangslage (b) befindet sich der Deckel 41' der untersten Mikrotiterplatte 15' bereits auf den Greifern 37 während der Behälter 43' der untersten Mikrotiterplatte 15' durch die Bolzen 39 der Kraftschluss-Verbindung fixiert ist. Die Transportvorrichtung 30 wird wiederum unterhalb des Behälters 43' der untersten Mikrotiterplatte 15' bewegt. Daraufhin kann die Kraftschluss-Verbindung durch Bewegen der Bolzen 39 nach aussen gelöst werden. Der Behälter 43' der untersten Mikrotiterplatte liegt auf der Transportvorrichtung 30 wie im Prozessschritt II) gezeigt.

Nach erfolgter Bearbeitung des Behälters 43' der untersten Mikrotiterplatte 15' wird dieser mit der Transportvorrichtung 30 zum Stapel zurückgeführt. Der Behälter 43' wird unterhalb des Deckels 41' der untersten Mikrotiterplatte 15' derart platziert, dass der Deckel 41' bündig auf dem Behälter 43' zu liegen kommt. Daraufhin werden die Greifer 37 nach aussen bewegt, wodurch sich die Formschluss-Verbindung mit dem Deckel 41' der untersten Mikrotiterplatte 15' löst und die gesamte Gewichtskraft des Stapels auf der Transportvorrichtung 30 liegt. Dieser Zustand ist im Prozessschritt III) dargestellt.

Die Bearbeitung der untersten Mikrotiterplatte 15' ist abgeschlossen und sie wird zusammen als Behälter 43' und Deckel 41' abtransportiert. Die sich unterhalb des Behälters 43' der untersten Mikrotiterplatten 15' befindende Transportvorrichtung 30 bewegt sich soweit nach unten, dass die Greifer 37 nun zwischen der unteren Kante des Behälters 43" und der unteren Kante des Deckels 41" der zweituntersten Mikrotiterplatte 15" zu liegen kommen. Durch Bewegen der Greifer 37 nach innen und der Transportvorrichtung 30 weiter nach unten liegt der Deckel 41" der zweituntersten Mikrotiterplatte 15" auf den Greifern 37 auf. Auf der Transportvorrichtung 30 bleiben somit die unterste Mikrotiterplatte 15' und der Behälter 43" der zweituntersten Mikrotiterplatte 15" wie im Prozessschritt IV) abgebildet.

Der Behälter 43" der zweituntersten Mikrotiterplatte 15" wird durch die Bolzen 39 des zweiten Haltewerkzeugs 35 fixiert. Dafür werden die Bolzen 39 nach innen bewegt, wenn der Behälter 43" auf deren Höhe sich befindet, siehe Prozessschritt V). Durch Fortbewegen der Transportvorrichtung 30 bleibt lediglich die unterste Mikrotiterplatte 15' auf der Transportvorrichtung 30 liegen. Diese liegt im bereits bearbeiteten Zustand vor und kann ihrer Anwendung entsprechend weggeführt werden. Die zweitunterste Mikrotiterplatte 15" befindet sich nun in jener Stellung, welche die unterste Mikrotiterplatte 15' in der zweiten Ausgangssituation (b) im Prozessschritt I) eingenommen hat.

Die erfindungsgemässe Vorrichtung 11 kann in zweifacher Ausführung in einem System mit nur einer Transportvorrichtung 30 angeordnet sein. Dabei können mit der gleichen Transportvorrichtung einem ersten Turm 13 vorzugsweise leere Mikrotiterplatten 15 entnommen und einem zweiten Turm 13 diese wieder im gefüllten Zustand zugeführt werden.

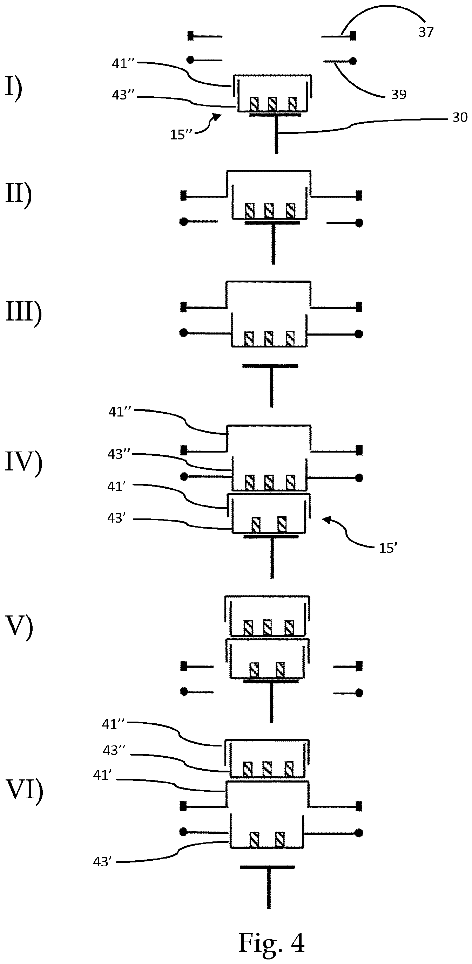

In Figur 4 ist schematisch ein Prozess für das Stapeln von Mikrotiterplatten 15 in einer erfindungsgemässen Vorrichtung 11 gezeigt. Der Prozess ist in sechs Schritte aufgeteilt, welche im Folgenden detailliert beschrieben werden.

Im ersten Prozessschritt I) ist der Turm 13 leer und auf der Transportvorrichtung 30 ist eine gefüllte Mikrotiterplatte 15" platziert. Die Transportvorrichtung 30 bewegt sich mit der Mikrotiterplatte 15" soweit nach oben und somit in den Turm 13 hinein bis der untere Rand des Deckels 41" der Mikrotiterplatte höher liegt als die Greifer 37 des oberen Haltewerkzeugs 33. Anschliessend werden die Greifer 37 ausgefahren. Bei der Bewegung der Transportvorrichtung 30 nach unten bleibt der Deckel 41" der Mikrotiterplatte auf den Greifern 37 liegen. Der Deckel 41" der Mikrotiterplatte ist durch eine Formschluss-Verbindung an seiner Position fixiert, wie im Prozessschritt II gezeigt. Wenn die Transportvorrichtung 30 sich weiter nach unten bewegt hat, sodass der Behälter 43" der Mikrotiterplatte auf der Höhe des unteren Haltewerkzeugs 35 liegt, fahren die Bolzen 39 des unteren Haltewerkzeugs 35 aus. Die Bolzen 39 fixieren den Behälter 43" der Mikrotiterplatte 15" mithilfe einer Kraftschluss-Verbindung. Somit liegen auf der Transportvorrichtung 30 keine Mikrotiterplatten und die Transportvorrichtung 30 ist für die Aufnahme einer weiteren Mikrotiterplatte 15 frei. Dieser Zustand ist im Prozessschritt III) abgebildet. Eine neu gefüllte Mikrotiterplatte 15' liegt auf der Transportvorrichtung 30 bereit zum Stapeln vor. Die Bolzen 39 ermöglichen aufgrund der seitlichen Krafteinwirkung auf den Behälter 43" der Mikrotiterplatte einen freien Zugang auf dessen gesamte Unterseite. Aus diesem Grund kann die Transportvorrichtung 30 mit der Mikrotiterplatte 15' soweit nach oben bewegt werden, dass die Oberseite des Deckels 41' der unteren Mikrotiterplatte 15' mit der Unterseite des Behälters 43" der oberen Mikrotiterplatte 15" in Berührung kommt. Das Einfahren der Bolzen 39 und das zeitgleiche Aufheben der Kraftschluss-Verbindung kann vorgenommen werden, ohne eine Änderung der Position des Behälters 43" der oberen Mikrotiterplatte 15" zu verursachen. Dieser Zustand ist im Prozessschritt IV) dargestellt. Bei Fortsetzen der Bewegung der Transportvorrichtung 30 nach oben kommt der Behälter 43" der oberen Mikrotiterplatte 15" in Kontakt mit dem Deckel 43", welcher auf den Greifern 37 aufliegt. Nach Erstellen eines Kontakts werden die Greifer 37 eingefahren. Auch durch das Einfahren der Greifer 37 wird keine Bewegung einer Mikrotiterplatte 15 verursacht. Die Transportvorrichtung 30 wird weiter nach oben bewegt und zwar so weit, dass der untere Rand des Deckels 41' der unteren Mikrotiterplatte 15' oberhalb der Bolzen 39 zu liegen kommt, wie im Prozessschritt V) gezeigt. Anschliessend wird die Transportvorrichtung 30 wieder nach unten bewegt und für das Fixieren des Deckels 41' und des Behälters 43' der unteren Mikrotiterplatte 15' die bereits oben beschriebenen Vorgänge aus den Schritten II) und III) befolgt. Als Ergebnis davon liegt der Deckel 41' der unteren Mikrotiterplatte 15' auf den Greifern 37 während der Behälter 43' der unteren Mikrotiterplatte 15' durch die Bolzen 39 mit einer Kraftschluss-Verbindung fixiert ist. Dieser Zustand ist im Prozessschritt VI) gezeigt und ist kongruent zu demjenigen im Prozessschritt III). Entsprechend können für das weitere Stapeln von Mikrotiterplatten 15 die Vorgänge in den Prozessschritten III) bis VI) beliebig oft wiederholt werden.

Eine erfindungsgemässe Vorrichtung kann ebenfalls Mikrotiterplatten stapeln, welche jeweils nur einen Behälter 43 und keinen Deckel umfassen. Falls die Greifer 37 die unterste Mikrotiterplatte 15' fixieren, liegt dabei die zweitunterste Mikrotiterplatte 15" auf den Bolzen 39 auf.

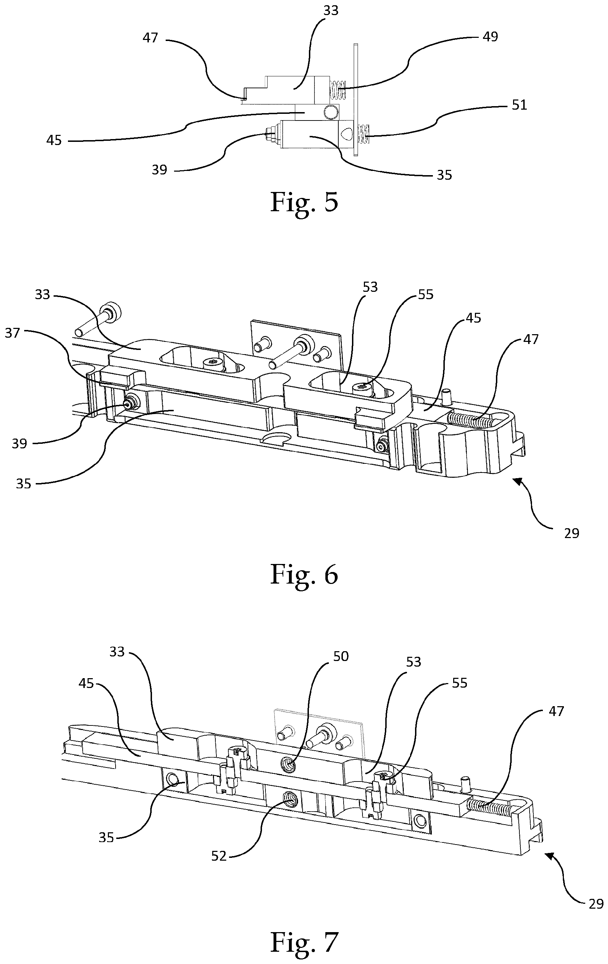

In Figur 5 ist ein Ausschnitt durch einen Backen 29 der Haltevorrichtung gezeigt, in welchem unter anderem das obere und das untere Haltewerkzeug 33,35 in deren Gesamtheit ersichtlich sind. Zwischen den beiden Haltewerkzeugen 33,35 ist ein Schiebebalken 45 angeordnet. Das obere und untere Haltewerkzeug 33,35 sind über je eine Feder 49,51 an der Haltevorrichtung 27 angebracht. Die Bewegung des Schiebebalkens 45 löst die Bewegung der beiden Haltewerkzeuge 33,35 aus. Der Schiebebalken 45 steht derart mit den beiden Haltewerkzeugen 33,35 in Verbindung, dass zuerst die Bewegung des oberen Haltewerkzeugs 33 ausgelöst wird. Die Bewegung des unteren Haltewerkzeuges 35 kann lediglich dann ausgeführt werden, wenn das obere Haltewerkzeug 33 sich bereits bewegt hat und sich in seiner ausgefahrenen Position befindet. Die an den Haltewerkzeugen 33,35 angeordneten Federn 49,51 sind gespannt, wenn die Haltewerkzeuge 33,35 nicht ausgefahren sind und sich in ihrer Ausgangsposition befinden.

Ein Backen der Haltevorrichtung 29 mit den beiden Haltewerkzeugen 33,35 und dem Schiebebalken 45 ist in den Figuren 6 und 7 dargestellt. Das obere Haltewerkzeug 33 weist an seiner Vorderseite zwei Greifer 37 auf. Diese sind an den Enden der Längsseite des oberen Haltewerkzeuges 33 angeordnet, so dass ein maximaler Abstand zwischen den Greifern 37 zustande kommt. Im oberen Haltewerkzeug 33 sind zwei Aussparungen 53 angeordnet, welche eine durchgehende Öffnung von der Oberseite zur Unterseite des Haltewerkzeugs 33 bilden. In diesen Aussparungen 53 kommt je ein Kulissenstein 55 zu liegen, welcher am Schiebebalken 45 angebracht ist, siehe Figur 7. Die Aussparungen 53 weisen jeweils eine kulissenartige Form auf, so dass durch Bewegen des Schiebebalkens 45 sich die relative Position des Kulissensteins 55 in Querrichtung zum Schiebebalken 45 gegenüber dem Haltewerkzeug 33 verändert. Die Änderung der relativen Position des Kulissensteins 55 gegenüber dem oberen Haltewerkzeug 33 ermöglicht es dem oberen Haltewerkzeug sich in Querrichtung zum Schiebebalken zu bewegen. Diese Bewegung wird durch die Feder 49 (nicht gezeigt), welche in nicht ausgefahrenem Zustand des Haltewerkzeugs unter Spannung steht, forciert.

Der Schiebebalken 45 ist über ein Federstössel 47 an der Haltevorrichtung 27 angebracht. Die Feder der Federstössel 47 ist eine Druckfeder, so dass im eingefahrenen Zustand der Haltewerkzeuge 33,35 die Feder des Federstössels 47 gespannt ist.

Die Kulissensteine 55 für das obere Haltewerkzeug 33 sind an der Oberseite des Schiebebalkens 45 angebracht. An der Unterseite des Schiebebalkens 45 sind Kulissensteine 55 für das untere Haltewerkzeug 35 angebracht. Diese sind in Längsrichtung des Schiebebalkens 45 versetzt zu denjenigen an der Oberseite angeordnet. Das untere Haltewerkzeug 35 weist ebenfalls zwei Aussparungen 53 auf, welche eine durchgehende Öffnung von dessen Oberseite zu dessen Unterseite bilden. Die Gestalt dieser Aussparungen 53 ist im Wesentlichen die gleiche wie diejenigen im oberen Haltewerkzeug 33, jedoch sind diese Aussparungen 53 im unteren Haltewerkzeug 35 gegenüber denjenigen im oberen Haltewerkzeug 33 in Längsrichtung des Schiebebalkens 45 versetzt angeordnet.

Aufgrund der versetzten Anordnung der Aussparungen 53 im oberen und unteren Haltewerkzeug 33,35 und der Kulissensteine 55 auf der Ober- und Unterseite des Schiebebalkens 45 kommt beim Bewegen des Schiebebalkens 45 eine sequentielle Bewegung zwischen derjenigen des unteren Haltewerkzeugs 35 und derjenigen des oberen Haltewerkzeugs 33 zustande. In der in Figur 6 abgebildeten Anordnung der Haltevorrichtung 27 muss das obere Haltewerkzeug 33 sich zwingend bewegt haben, bevor das untere Haltewerkzeug 35 sich bewegen kann. In Abhängigkeit der Auslenkung des Schiebebalkens 45 kann auch nur das obere Haltewerkzeug 33 hin und her bewegbar sein.

In Figur 7 ist die Bohrung 50,52 für die Führung der Federn 49,51, über welche die Haltewerkzeuge 33,35 an der Haltevorrichtung 27 angebracht sind, dargestellt. Die Bohrungen 50,52 sind jeweils etwa in der Mitte der Haltewerkzeuge 33,35 angebracht, damit kein Drehmoment beim Ausfahren der Feder 49,51 entsteht und die zwei Greifer 37 bzw. zwei Bolzen 39 des Haltewerkzeugs 33,35 stets gleichzeitig bewegt werden.

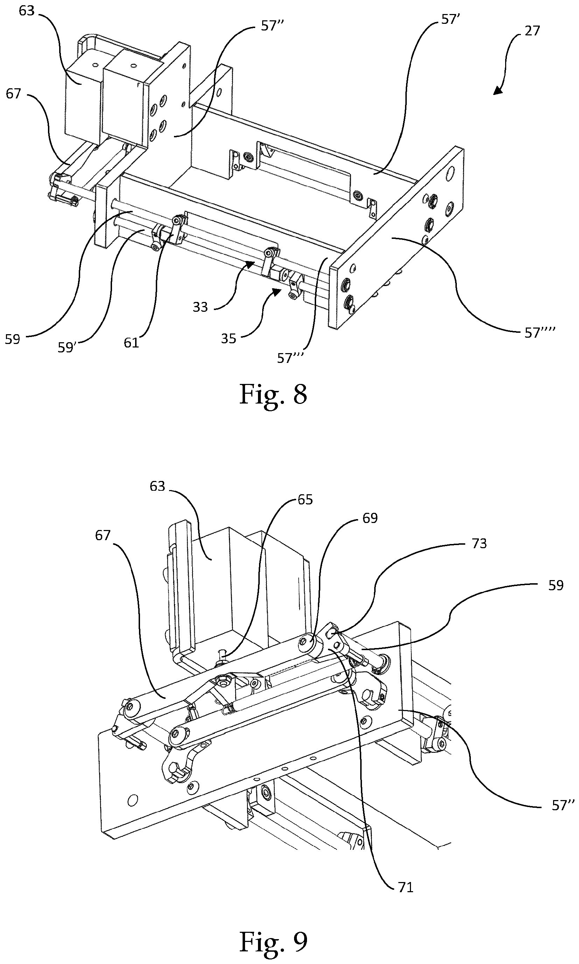

In Figuren 8 und 9 ist eine alternative Haltevorrichtung 27 gezeigt. Die Haltevorrichtung 27 weist 4 Wände auf, welche alle vier Seiten des unteren Endes des Turms (nicht gezeigt) umschliessen. An zwei gegenüberliegenden Wänden 57',57‴ der Haltevorrichtung 27 sind Haltewerkzeuge 33, 35 angebracht. Diese Wände weisen Aussparungen auf, um die Haltewerkzeuge 33,35 durch diese Aussparungen von der Aussenseite in die Innenseite der Haltevorrichtung 27 zu führen. Die Wände 57',57" sind an den weiteren zwei Wänden 57",57"" der Haltevorrichtung mit Hilfe von Schrauben angebracht, so dass die Wände 57',57'" von einer Wand 57" bis zur anderen Wand 57"" reichen. Die Wände 57",57"" reichen über die Kanten der Wände 57',57‴ hinaus und weisen an diesen Stellen jeweils zwei Durchbohrungen auf. Diese Durchbohrungen sind derart angeordnet, dass zwei Rundstäbe 59, 59' durch die vier Durchbohrungen geführt sind, so dass die Rundstäbe 59, 59' übereinander zu liegen kommen und in den Durchbohrungen drehbar gelagert sind. An den Rundstäben 59, 59' sind die Haltewerkzeuge 33,35 angebracht, wobei jedes Haltewerkzeug zwei an einem Rundstab fest angebrachte Haltekörper 61 und wiederum an die Haltekörper 61 angebrachte Stifte aufweist. Die Stifte bilden jenen Teil der Haltewerkzeuge 33, 35, welche in die Innenseite der Haltevorrichtungen geführt werden können.

An einer Wand 57", welche senkrecht zu den beiden Wänden 57',57‴ mit den Haltevorrichtungen angeordnet ist, ist der Antrieb für die Bewegung der Rundstäbe 59 und somit der Haltewerkzeuge 33, 35 angebracht. Der Antrieb umfasst zwei Aktuatoren 63, welche über eine Kipphebel-Anordnung die Rotation der Rundstäbe 59,59' bewirken. Der erste Aktuator 63 ist in der Mitte der Wand 57" und der zweite Aktuator an der der Wand 57" abgewandten Seite des ersten Aktuators angebracht. Beide Aktuatoren weisen einen Stössel 65 auf, welcher in Stapelrichtung der Vorrichtung 11 nach unten bewegbar ist. Der Stössel 65 bildet jeweils die Verbindung zwischen dem Aktuator 63 und einer dem Stössel 65 senkrecht angeordneten Übertragungsstange 67, welche an beiden Enden eine Bohrung aufweist. Durch die Bohrungen an den Übertragungsstangen 67 sind Bolzen 69 geführt, welche die Übertragungsstangen 67 an ihren Enden mit einem Kipphebel 71 verbinden. Der Stössel 65 ist in der Mitte der Übertragungsstange 67 angebracht, so dass die Bewegung des Stössels 65 kein Drehmoment auf der Übertragungsstange 67 verursacht. Der Kipphebel 71 weist neben einer Bohrung 75 für die Aufnahme des Bolzens eine maulschlüsselförmige Aussparung 73 auf. Diese Aussparung dient der Aufnahme eines Rundstabs 59. Die Rundstäbe 59 haben an ihren Enden, welche auf der Seite der Aktuatoren 63 zu liegen kommen, einen mehrkantigen Rand. Der mehrkantige Rand ermöglicht es, die bei einer Drehbewegung des Kipphebels 71 entstehende Torsionskraft auf den Rundstab 59 zu übertragen. Die maulschlüsselförmige Aussparung 73 weist eine feste Verbindung mit dem Rundstab 59 auf, wodurch bei einer Bewegung des Bolzens 69 in der Bohrung 75 der Kipphebel 71 lediglich eine Rotation um den Rundstab 59 durchführt und gleichzeitig den Rundstab 59 rotiert.

Während vorstehend spezifische Ausführungsformen beschrieben wurden, ist es offensichtlich, dass unterschiedliche Kombinationen der aufgezeigten Ausführungsmöglichkeiten angewendet werden können, insoweit die Ausführungsmöglichkeiten nicht vom Gegenstand der Ansprüche abweichen.

### BEZUGSZEICHENLISTE:

- 11: Vorrichtung
- 13: Turm
- 15: Mikrotiterplatten
- 17: Stange
- 19: U-Förmiges Bauteil
- 21: Bewegliche Stange
- 23: Verbindungselemente
- 25: Vorderseite des Turms
- 27: Haltevorrichtung
- 29: Backen für Haltevorrichtung
- 30: Transportvorrichtung
- 31: Mittelstück der Haltevorrichtung
- 33: Oberes Haltewerkzeug
- 35: Unteres Haltewerkzeug
- 37: Greifer
- 39: Bolzen
- 41: Deckel der Mikrotiterplatte
- 43: Behälter der Mikrotiterplatte
- 45: Schiebebalken
- 47: Federstössel/Federelement
- 49: Feder am oberen Haltewerkzeug
- 50: Bohrung für die Feder im oberen Haltewerkzeug
- 51: Feder am unteren Haltewerkzeug
- 52: Bohrung für die Feder im unteren Haltewerkzeug
- 53: Aussparung im Haltewerkzeug
- 55: Kulissenstein
- 57: Wand
- 59: Rundstab
- 61: Haltekörper
- 63: Aktuator
- 65: Stössel
- 67: Übertragungsstange
- 69: Bolzen in der Übertragungsstange
- 71: Kipphebel
- 73: Maulschlüsselförmige Aussparung
- 75: Bohrung

## Patentansprüche

1. Vorrichtung (11) zum Aufnehmen, Stapeln und Entnehmen von Mikrotiterplatten (15) umfassend;
einen Turm (13) für die Stapelung von Mikrotiterplatten (15), wobei eine Mikrotiterplatte einen Behälter (43) und optional einen Deckel (41) umfasst,
eine am unteren Ende des Turms angeordnete Haltevorrichtung (27), welche ein erstes (33) und ein zweites Haltewerkzeug (35) aufweist und vorzugsweise vorgesehen ist, eine Mikrotiterplatte (15) teilweise zu umschliessen,
wobei das erste Haltewerkzeug (33) ausgebildet ist, eine Mikrotiterplatte (15) formschlüssig zu halten, und
das erste und zweite Haltewerkzeug (33,35) zur Mitte des Turms (13) hin bewegbar an der Haltevorrichtung (27) angeordnet sind,
**dadurch gekennzeichnet, dass**
das erste Haltewerkzeug (33) oberhalb des zweiten Haltewerkzeugs (35) angeordnet ist, und
das zweite Haltewerkzeug (35) ausgebildet ist, den Behälter (43) der Mikrotiterplatte kraftschlüssig zu fixieren.

2. Vorrichtung (11) gemäss Anspruch 1, **dadurch gekennzeichnet, dass** am ersten Haltewerkzeug (33) ein Greifer (37) angebracht ist und der Greifer (37) geeignet ist, die Mikrotiterplatte (15) formschlüssig zu halten und vorzugsweise der Greifer (37) im ausgefahrenen Zustand etwa bis zur Seitenwand des Behälters (43) einer im Turm (13) aufgenommenen Mikrotiterplatte (11) reicht.

3. Vorrichtung (11) gemäss Anspruch 2, **dadurch gekennzeichnet, dass** am ersten Haltewerkzeug (33) zwei Greifer (37) angebracht sind.

4. Vorrichtung (11) gemäss einem der Ansprüche 1 bis 3, **dadurch gekennzeichnet, dass** am zweiten Haltewerkzeug (35) ein Bolzen (39) angebracht ist und der Bolzen (39) vorgesehen ist, den Behälter (43) der Mikrotiterplatte zu fixieren.

5. Vorrichtung (11) gemäss Anspruch 4, **dadurch gekennzeichnet, dass** am zweiten Haltewerkzeug (35) zwei Bolzen (39) angebracht sind.

6. Vorrichtung (11) gemäss einem der Ansprüche 1 bis 5, **dadurch gekennzeichnet, dass** das erste Haltewerkzeug (33) über ein erstes Federelement (49) an der Haltevorrichtung (27) angeordnet ist, wobei das erste Federelement (49) vorzugsweise eine Druckfeder umfasst.

7. Vorrichtung (11) gemäss einem der Ansprüche 1 bis 6, **dadurch gekennzeichnet, dass** das zweite Haltewerkzeug (35) über ein zweites Federelement (51) an der Haltevorrichtung (27) angeordnet ist, wobei das zweite Federelement (51) vorzugsweise eine Druckfeder umfasst.

8. HaltewerkVorrichtung (11) gemäss einem der Ansprüche 1 bis 7, **dadurch gekennzeichnet, dass** ein Schiebebalken (45) an der Haltevorrichtung (27) angebracht ist, welcher die Bewegungen der beiden Haltewerkzeuge (33,35) auslöst.

9. Vorrichtung (11) gemäss Anspruch 8, **dadurch gekennzeichnet, dass** der Schiebebalken (45) derart an der Haltevorrichtung (27) angeordnet ist und mit den beiden Haltewerkzeugen (33,35) in Verbindung steht, dass der Schiebebalken (45) in einem ersten Schritt die Bewegung des ersten Haltewerkzeugs (33) und in einem zweiten Schritt die Bewegung des zweiten Haltewerkzeugs (35) auslöst.

10. Vorrichtung (11) gemäss einem der Ansprüche 1 bis 9, **dadurch gekennzeichnet, dass** die Haltevorrichtung (27) je zwei erste und zweite Haltewerkzeuge (33,35) aufweist, welche jeweils gegenüberliegend zueinander angeordnet sind.

11. System mit einer Vorrichtung (11) zum Aufnehmen, Stapeln und Entnehmen von Mikrotiterplatten (13) gemäss einem der Ansprüche 1 bis 10, einer Dispensier-Vorrichtung zum Befüllen von Mikrotiterplatten (15) und einer Transportvorrichtung (30) zur Entnahme und zum Zuführen von Mikrotiterplatten aus der oder zur Vorrichtung (11).

12. System nach Anspruch 11 mit einer ersten und einer zweiten Vorrichtung (11) zum Aufnehmen, Stapeln und Entnehmen von Mikrotiterplatten, welche eine gemeinsame Transportvorrichtung (30) aufweisen.

13. Verfahren zum Hinzufügen von Mikrotiterplatten (15) zu einem Stapel von Mikrotiterplatten oder Entnehmen von Mikrotiterplatten aus einem Stapel Mikrotiterplatten, wobei der Stapel sich in z-_Richtung erstreckt, bei welchem Verfahren eine Mikrotiterplatte (15) jeweils von unten her zum Stapel hinzugefügt respektive die unterste Mikrotiterplatte (15') des Stapels entnommen wird, wobei die unterste und zweitunterste Mikrotiterplatte (15', 15") jeweils durch in einer Ebene beweglichen und übereinander angeordneten Haltewerkzeugen (33,35) gehalten werden, während eine in mindestens zwei Richtungen bewegliche Transportvorrichtung (30) eine neue Mikrotiterplatte (15) von unten dem Stapel hinzufügt oder die unterste Mikrotiterplatte (15') dem Stapel entnimmt, wobei die Mikrotiterplatten in Z-Richtung gestapelt werden,
**dadurch gekennzeichnet, dass**
das obere Haltewerkzeug eine formschlüssige Verbindung und das untere Haltewerkzeug eine kraftschlüssige Verbindung mit der jeweiligen Mikrotiterplatte eingeht.

14. Verfahren nach Anspruch 13, **dadurch gekennzeichnet, dass** bei der Entnahme einer Mikrotiterplatte (15) aus dem Stapel
a) die Transportvorrichtung (30) unter die unterste Mikrotiterplatte (15') positioniert wird,
b) die kraftschlüssige Verbindung zwischen der untersten Mikrotiterplatte (15') und des unteren Haltewerkzeugs (35) gelöst wird, so dass die unterste Mikrotiterplatte (15') auf der Transportvorrichtung (30) aufliegt,
c) die Transportvorrichtung (30) in Z-Richtung so weit nach oben bewegt wird, bis die auf der Transportvorrichtung aufliegende unterste Mikrotiterplatte (15') mit der zweituntersten Mikrotiterplatte (15") Kontakt hat,
d) die formschlüssige Verbindung zwischen der zweituntersten Mikrotiterplatte (15") und des oberen Haltewerkzeugs (33) gelöst wird, so dass der Stapel von Mikrotiterplatten auf der Transportvorrichtung (30) aufliegt,
e) die Transportvorrichtung (30) in Z-Richtung nach unten bewegt wird bis die drittunterste Mikrotiterplatte (15‴) auf der Höhe des oberen Haltewerkzeugs zu liegen kommt,
f) die drittunterste Mikrotiterplatte (15‴) durch das obere Haltewerkzeug (33) formschlüssig gehalten wird,
g) die Transportvorrichtung in Z-Richtung weiter nach unten bewegt wird, bis die zweitunterste Mikrotiterplatte (15") auf der Höhe des unteren Haltwerkzeugs zu liegen kommt,
h) sodass die zweitunterste Mikrotiterplatte (15") durch das untere Haltewerkzeug (35) kraftschlüssig in Eingriff genommen wird,, und
i) die Transportvorrichtung (30) mit der untersten Mikrotiterplatte (15') weggeführt wird,
und beim Hinzufügen einer Mikrotiterplatte (15) zum Stapel
j) eine auf der Transportvorrichtung (30) aufliegende Mikrotiterplatte unter die unterste Mikrotiterplatte (15') im Stapel positioniert wird,
k) die kraftschlüssige Verbindung zwischen der untersten Mikrotiterplatte (15') und dem unteren Haltewerkzeug (35) gelöst wird, so dass die unterste Mikrotiterplatte (15') auf der auf der Transportvorrichtung aufliegenden Mikrotiterplatte (15) aufliegt,
l) die Transportvorrichtung (30) in Z-Richtung so weit nach oben bewegt wird, bis die vormals unterste Mikrotiterplatte (15') mit der darüberliegenden zweituntersten Mikrotiterplatte (15") Kontakt hat,
m) die formschlüssige Verbindung zwischen der vormals zweituntersten Mikrotiterplatte (15") und dem oberen Haltewerkzeug (33) gelöst wird, so dass die vormals zweitunterste Mikrotiterplatte (15") auf der untersten Mikrotiterplatte (15') aufliegt,
n) die Transportvorrichtung (30) in Z-Richtung nach oben bewegt wird, bis die vormals unterste Mikrotiterplatte (15') des Stapels auf der Höhe des oberen Haltewerkzeugs (33) zu liegen kommt,
o) die vormals unterste Mikrotiterplatte (15') durch das obere Haltewerkzeug (33) formschlüssig in Eingriff genommen wird,
p) die Transportvorrichtung (30) in Z-Richtung nach unten bewegt wird, bis die auf der Transportvorrichtung liegende Mikrotiterplatte (15) auf der Höhe des unteren Haltewerkzeugs (35) zu liegen kommt,
q) die auf der Transportvorrichtung (30) liegende Mikrotiterplatte (15) durch das untere Haltewerkzeug (35) kraftschlüssig in Eingriff genommen werden kann,

15. Verfahren nach Anspruch 13, **dadurch gekennzeichnet, dass** im Falle von Mikrotiterplatten (15), die je einen Behälter (43) und einen Deckel (41) umfassen,
bei der Entnahme einer Mikrotiterplatte (15) aus dem Stapel
- in den Verfahrensschritten e) und f) der Deckel (41") der zweituntersten Mikrotiterplatte durch das obere Haltewerkzeug (33) gehalten wird, und
- in den Verfahrensschritten g) und h) der Behälter (43") der zweituntersten Mikrotiterplatte durch das untere Haltewerkzeug (35) gehalten wird, und
beim Hinzufügen einer Mikrotiterplatte (15) zum Stapel
- die auf der Transportvorrichtung (30) aufliegende Mikrotiterplatte (15) einen Behälter (43) und einen Deckel (41) umfasst,
- in den Verfahrensschritten o) und p) der Deckel (41) der auf der Transportvorrichtung liegende Mikrotiterplatte durch das obere Haltewerkzeug (33) gehalten wird, und
- im Verfahrensschritt r) der Behälter (43) der auf der Transportvorrichtung liegenden Mikrotiterplatte durch das untere Haltewerkzeug (35) gehalten wird.

## Claims

1. Device (11) for receiving, stacking and removing microtiter plates (15) comprising:
a tower (13) for stacking microtiter plates (15), wherein a microtiter plate comprises a container (43) and optionally a lid (41),
a holding device (27) arranged at the lower end of the tower that has a first (33) and a second holding tool (35) and that is preferably provided to partially enclose a microtiter plate (15),
wherein the first holding tool (33) is configured to positively hold a microtiter plate (15) and
the first and the second holding tool (33, 35) are arranged on the holding device (27) movable towards the middle of the tower (13),
**characterized in that**
the first holding tool (33) is arranged above the second holding tool (35) and
the second holding tool (35) is configured to frictionally fix the container (43) of the microtiter plates.

2. Device (11) according to claim 1, **characterized in that** a gripper (37) is mounted on the first holding tool (33) and the gripper (37) is appropriate to positively hold the microtiter plate (15) and preferably the gripper (37) in the extended state reaches up to the side wall of the container (43) of a microtiter plate (11) received in the tower (13).

3. Device (11) according to claim 2, **characterized in that** two grippers (37) are mounted on the first holding tool (33).

4. Device (11) according to one of the claims 1 to 3, **characterized in that** a bolt (39) is mounted on the second holding tool (35) and the bolt (39) is provided to fix the container (43) of the microtiter plate.

5. Device (11) according to claim 4, **characterized in that** two bolts (39) are mounted on the second holding device (35).

6. Device (11) according to one of the claims 1 to 5, **characterized in that** the first holding tool (33) is arranged via a first spring element (49) on the holding device (27), wherein the first spring element (49) preferably comprises a compression spring.

7. Device (11) according to one of the claims 1 to 6, **characterized in that** the second holding tool (35) is arranged via a second spring element (51) on the holding device (27), wherein the second spring element (51) preferably comprises a compression spring.

8. Device (11) according to one of the claims 1 to 7, **characterized in that** a slider bar (45) is mounted on the holding device (27) that triggers the movements of the two holding tools (33, 35).

9. Device (11) according to claim 8, **characterized in that** the slider bar (45) is arranged on the holding device (27) and is connected to the two holding tools (33, 35) so that the slider bar (45) triggers in a first step the movement of the first holding tool (33) and in a second step the movement of the second holding tool (35).

10. Device (11) according to one of the claims 1 to 9, **characterized in that** the holding device (27) has respectively two first and second holding tools (33, 35) that are arranged respectively facing one another.

11. System with a device for receiving, stacking and removing microtiter plates (15) according to one of the claims 1 to 10, a dispensing device for filling microtiter plates (15) and a conveying device (30) for removing and supplying microtiter plates from or to the device (11).

12. System according to claim 11 with a first and a second device (11) for receiving, stacking and removing microtiter plates that have a common conveying device (30).

13. Method for adding microtiter plates (15) to a stack of microtiter plates or removing microtiter plates from a stack of microtiter plates, wherein the stack extends in z direction, for which method a microtiter plate (15) is added to the stack respectively from below or the lowest microtiter plate (15') of the stack is removed, wherein the lowest and the second lowest microtiter plate (15', 15") are held respectively by holding tools (33, 35) movable in a plane and arranged above one another, while a conveying device (30) movable in at least two directions adds a new microtiter plate (15) from below to the stack or the lowest microtiter plate (15') is removed from the stack, whereby the microtiter plates are stacked in z direction,
**characterized in that**
the upper holding tool enters into a positive connection and the lower holding tool enters a frictional connection with the respective microtiter plate.

14. Method according to claim 13, **characterized in that**, when removing a microtiter plate (15) from the stack,
a) the conveying device (30) is positioned below the lowest microtiter plate (15'),
b) the frictional connection between the lowest microtiter plate (15') and the lower holding tool (35) is released so that the lowest microtiter plate (15') rests on the conveying device (30),
c) the conveying device (30) is moved in z direction upwards until the lowest microtiter plate (15') that rests on the conveying device contacts the second lowest microtiter plate (15"),
d) the positive connection between the second lowest microtiter plate (15") and the upper holding tool (33) is released so that the stack of microtiter plates rests on the conveying device (30),
e) the conveying device (30) is moved downwards in z direction until the third lowest microtiter plate (15‴) comes to rest at the level of the upper holding tool,
f) the third lowest microtiter plate (15‴) is positively held by the upper holding tool (33),
g) the conveying device is further moved downwards in z direction until the second lowest microtiter plate (15") comes to rest at the level of the lower holding tool,
h) so that the second lowest microtiter plate (15") is frictionally engaged by the lower holding tool (35) and
i) the conveying device (30) is taken away with the lowest microtiter plate (15')
and, when adding a microtiter plate (15) to the stack,
j) a microtiter plate resting on the conveying device (30) is positioned below the lowest microtiter plate (15') in the stack,
k) the frictional connection between the lowest microtiter plate (15') and the lower holding tool (35) is released so that the lowest microtiter plate (15') rests on the microtiter plate resting on the conveying device,
I) the conveying device (30) is further moved upwards in z direction until the previously lowest microtiter plate (15') contacts the overlying second lowest microtiter plate (15"),
m) the positive connection between the previously second lowest microtiter plate (15") and the upper holding tool (33) is released so that the previously second lowest microtiter plate (15") rests on the lowest microtiter plate (15'),
n) the conveying device (30) is moved upwards in z direction until the previously lowest microtiter plate (15') of the stack comes to rest at the level of the upper holding tool (33),
o) the previously lowest microtiter plate (15') is positively engaged by the upper holding tool (33),
p) the conveying device (30) is moved downwards in z direction until the microtiter plate (15) lying on the conveying device comes to rest at the level of the lower holding tool (35),
q) the microtiter plate (15) lying on the conveying device (30) can be frictionally engaged by the lower holding tool (35).

15. Method according to claim 13, **characterized in that** in the case of microtiter plates (15) that each comprise a container (43) and a lid (41), when removing a microtiter plate (15) from the stack,
- in the method steps e) and f) the lid (41") of the second lowest microtiter plate is held by the lower holding tool (33) and
- in the method steps g) and h) the container (43") of the second lowest microtiter plate is held by the lower holding tool (35) and,
when adding a microtiter plate (15) to the stack,
- the microtiter plate (15) resting on the conveying device (30) comprises a container (43) and a lid (41),
- in the method steps o) and p) the lid (41) of the microtiter plate lying on the conveying device is held by the upper holding tool (33) and
- in the method step r) the container (43) of the microtiter plate lying on the conveying device is held by the lower holding tool (35).

## Revendications

1. Dispositif (11) pour recevoir, empiler et prélever des plaques de microtitrage (15) comprenant :
une tour (13) pour l'empilage de plaques de microtitrage (15), cependant qu'une plaque de microtitrage comprend un récipient (43) et en option un couvercle (41),
un dispositif de support (27), placé à l'extrémité inférieure de la tour, qui présente un premier (33) et un second outil de support (35) et qui est de préférence prévu pour envelopper partiellement une plaqus de microtitrage (15),
cependant que le premier outil de support (33) est configuré pour maintenir par complémentarité de formes une plaque de microtitrage (15) et
le premier et le second outil de support (33, 35) sont placés sur le dispositif de support (27) en étant mobiles vers le milieu de la tour (13),
**caractérisé en ce que**
le premier outil de support (33) est placé au-dessus du second outil de support et
le second outil de support (35) est configuré pour fixer par adhérence le récipient (43) de la plaque de microtitrage (15).

2. Dispositif selon la revendication 1, **caractérisé en ce qu'**un préhenseur (37) est monté sur le premier outil de support (33) et le préhenseur (37) est approprié pour maintenir la plaque de microtitrage par complémentarité de formes et de préférence le préhenseur, à l'état déployé, s'étend approximativement jusqu'à la paroi latérale du récipient (43) d'une plaque de microtitrage (15) logée dans la tour (13).

3. Dispositif (11) selon la revendication 2, **caractérisé en ce que** deux préhenseurs (37) sont montés sur le premier outil de support (33).

4. Dispositif (11) selon l'une des revendications 1 à 3, **caractérisé en ce qu'**un boulon (39) est fixé sur le second outil de support (35) et le boulon (39) est prévu pour fixer le récipient (43) de la plaque de microtitrage (15).

5. Dispositif (11) selon la revendication 4, **caractérisé en ce que** deux boulons (39) sont fixés sur le second outil de support (35).

6. Dispositif (11) selon l'une des revendications 1 à 5, **caractérisé en ce que** le premier outil de support (33) est placé sur le dispositif de support (27) par un premier élément à ressort (49), le premier élément de ressort (49) comprenant de préférence un ressort de compression.

7. Dispositif (11) selon l'une des revendications 1 à 6, **caractérisé en ce que** le second outil de support (35) est placé sur le dispositif de support (27) par un second élément à ressort (51), le second élément de ressort (51) comprenant de préférence un ressort de compression.

8. Dispositif (11) selon l'une des revendications 1 à 7, **caractérisé en ce qu'**une barre coulissante (45) qui déclenche les mouvements des deux outils de support (33, 35) est montée sur le dispositif de support (27).

9. Dispositif (11) selon la revendication 8, **caractérisé en ce que** la barre coulissante (45) est placée sur le dispositif de support (27) de telle manière et est en relation avec les deux outils de support (33, 35) si bien que la barre coulissante (45) dans une première étape déclenche le mouvement du premier outil de support (33) et dans une seconde étape le mouvement du second outil de support (35).

10. Dispositif (11) selon l'une des revendications 1 à 9, **caractérisé en ce que** le dispositif de support (27) présente respectivement deux premiers et deux seconds outils de support (33, 35) qui sont placés respectivement en face l'un de l'autre.

11. Système avec un dispositif (11) pour recevoir, empiler et prélever des plaques de microtitrage (15) selon l'une des revendications 1 à 10, un dispositif de distribution pour remplir des plaques de microtitrage (15) et un dispositif de transport (30) pour prélever du dispositif (11) et amener au dispositif (11) des plaques de microtitrage (15).

12. Système selon la revendication 11 avec un premier et un second dispositif (11) pour recevoir, empiler et prélever des plaques de microtitrage qui présentent un dispositif de transport commun (30).

13. Procédé pour ajouter des plaques de microtitrage (15) à une pile de plaques de microtitrage ou prélever des plaques de microtitrage d'une pile de plaques de microtitrage, la pile s'étendant dans la direction z, procédé pour lequel une plaque de microtitrage (15) est ajoutée à la pile par le bas ou la plaque de microtitrage la plus basse (15') de la pile est prélevée, cependant que la plaque de microtitrage la plus basse et l'avant-dernière (15', 15") sont maintenues respectivement par des outils de support (33, 35) mobiles dans un plan et placés l'un au-dessus de l'autre, tandis qu'un dispositif de transport (30) mobile dans au moins deux directions ajoute une nouvelle plaque de microtitrage (15) à la pile par le bas ou prélève la plaque de microtitrage la plus basse (15') de la pile, les plaques de microtitrage étant empilées dans la direction z,
**caractérisé en ce que**
l'outil de support supérieur réalisé une jonction par complémentarité de formes et l'outil de support inférieur une jonction par adhérence avec la plaque de microtitrage respective.

14. Procédé selon la revendication 13, **caractérisé en ce que**, lors du prélèvement d'une plaque de microtitrage (15) de la pile
a) le dispositif de transport (30) est positionné sous la plaque de microtitrage la plus basse (15'),
b) la jonction par adhérence entre la plaque de microtitrage la plus basse (15') et l'outil de support inférieur (35) est desserrée de telle manière que la plaque de microtitrage la plus basse (15') repose sur le dispositif de transport (30),
c) le dispositif de transport (30) est déplacé dans la direction z vers le haut jusqu'à ce que la plaque de microtitrage la plus basse (15') qui repose sur le dispositif de transport soit en contact avec l'avant-dernière plaque de microtitrage (15"),
d) la jonction par complémentarité de formes entre l'avant-dernière plaque de microtitrage (15") et l'outil de support supérieur (33) est desserrée si bien que la pile de plaques de microtitrage repose sur le dispositif de transport (30),
e) le dispositif de transport (30) est déplacé dans la direction z vers le bas jusqu'à ce que l'avant-avant-dernière plaque de microtitrage (15‴) vienne reposer à hauteur de l'outil de support supérieur,
f) l'avant-avant-dernière plaque de microtitrage (15‴) est maintenue par complémentarité de formes par l'outil de support supérieur (33),
g) le dispositif de transport est encore déplacé dans la direction z vers le bas jusqu'à ce que l'avant-dernière plaque de microtitrage (15") vienne reposer à hauteur de l'outil de support inférieur
h) si bien que l'avant-dernière plaque de microtitrage (15") est mise en prise par adhérence par l'outil de support inférieur (35) et
i) le dispositif de transport (30) avec l'avant-dernière plaque de microtitrage (15") est éloigné
et, lors de l'ajout d'une plaque de microtitrage (15) à la pile
j) une plaque de microtitrage qui repose sur le dispositif de transport (30) est positionnée sous la plaque de microtitrage la plus basse (15'),
k) la jonction par adhérence entre la plaque de microtitrage la plus basse (15') et l'outil de support inférieur (35) est desserrée si bien que la plaque de microtitrage la plus basse (15') repose sur la plaque de microtitrage qui repose sur le dispositif de transport,
l) le dispositif de transport (30) est déplacé vers le haut dans la direction z jusqu'à ce que la plaque de microtitrage précédemment la plus basse (15') soit en contact avec l'avant-dernière plaque de microtitrage (15") située au-dessus,
m) la jonction par complémentarité de formes entre la précédemment avant-dernière plaque de microtitrage (15") et l'outil de support supérieur (33) est desserrée si bien que la précédemment avant-dernière plaque de microtitrage (15") repose sur la plaque de microtitrage la plus basse (15'),
n) le dispositif de transport (30) est déplacé vers le haut dans la direction z jusqu'à ce que la plaque de microtitrage précédemment la plus basse (15') de la pile vienne reposer au niveau de l'outil de support supérieur (33),
o) la plaque de microtitrage précédemment la plus basse (15') est mise en prise par complémentarité de formes par l'outil de support supérieur,
p) le dispositif de transport (30) est déplacé vers le bas dans la direction z jusqu'à ce que la plaque de microtitrage (15) située sur le dispositif de transport vienne reposer au niveau de l'outil de support inférieur (35),
q) la plaque de microtitrage (15) située sur le dispositif de transport (30) est mise en prise par adhérence par l'outil de support inférieur (35).

15. Procédé selon la revendication 13, **caractérisé en ce que**, dans le cas de plaques de microtitrage (15) qui comprennent chacune un récipient (43) et un couvercle (41),
lors du prélèvement d'une plaque de microtitrage (15) de la pile
- dans les étapes de procédé e) et f) le couvercle (41") de l'avant-dernière plaque est maintenu par l'outil de support supérieur (33) et
- dans les étapes de procédé g) et h) le récipien (43") de l'avant-dernière plaque est maintenu par l'outil de support inférieur (35) et
lors de l'ajout d'une plaque de microtitrage (15) à la pile
- la plaque de microtitrage (15) qui repose sur le dispositif de transport (30) comprend un récipient (43) et un couvercle (41),
- dans les étapes de procédé o) et p) le couvercle (41) de la plaque de microtitrage située sur le dispositif de transport est maintenu par l'outil de support supérieur (33) et
- dans l'étape de procédé r) le récipient de de la plaque de microtitrage située sur le dispositif de transport est maintenu par l'outil de support inférieur (35).
